# EUROPEAN PATENT APPLICATION

(11) **EP 4 814 972 A1**
(43) Date of publication of application: **30.09.2026**
(21) Application number: 25166142.7
(22) Date of filing: 25.03.2025
(51) Int. Cl.: G16C 20/50, G16C 20/70, G16C 20/20, G16C 20/30, G16B 40/20

(54) **GENERATION OF MOLECULAR STRUCTURES**

(71) Applicant: Robert Bosch GmbH, 70442 Stuttgart (DE)
(72) Inventor: van de Meent, Jan-Willem, 3523 KA Utrecht (NL); Tiemann, Michael, 72074 Tuebingen (DE); Hadzi Veljkovic, Tin, 1012 WP Amsterdam (NL)

(57) **Abstract**

Some embodiments are directed to generating molecular structures, which may include progressively denoising a spatial density function by iteratively applying a trained neural network. The neural network is trained to transform noisy spatial density functions into structured spatial density functions based on training data comprising molecular structures represented as spatial density functions. After denoising, a sum of predefined density distributions is fitted to the denoising result to obtain the molecular structure.

## Description

### Technical Field

The presently disclosed subject matter relates to a method for generating molecular structures, a method for training a neural network to transform noisy spatial functions into structured spatial density functions, a computer-readable medium, and a system.

### Background

Geometric graphs are typically processed using Graph Neural Networks (GNNs) for inference or generative tasks. In a generative setting, this implies that we need to specify the number of nodes in the graph beforehand, thus conditioning the network on this information.

Graphs are common structures that have found extensive applications in various domains, including computer science, social networks, and biological systems. Traditionally, graphs are represented as discrete objects, comprising of a finite set of nodes and edges connecting them. In this formalism, the existence of nodes and edges is binary - they are either present or not. In this work, we propose a novel perspective on graphs, treating them as continuous objects embedded in a high-dimensional space, typically Euclidean space. By extending the notion of node existence to a continuous domain, we can model the uncertainty and probabilistic nature of many real-world systems. This continuous graph representation, for example, is particularly relevant in the context of molecular modeling, where particles, such as electrons, inherently exhibit probabilistic behavior and do not have precisely defined positions due to quantum effects.

There is a need for a more versatile method to generate molecular structures.

### Summary

It would be advantageous to have an improved method for generating molecular structures. For example, a generated molecular structure may comprise a plurality of molecular substructure positions. Typically, a molecular structure is a molecule, and molecular substructures are atoms; however other examples are provided herein.

The method may comprise progressively denoising a spatial function by iteratively applying a trained neural network. The neural network is trained to transform noisy density functions into structured spatial density functions based on training data comprising molecular structures represented as spatial density functions. Initially, the spatial function may be generated as a noisy spatial function; however, during denoising, the spatial function is transformed into a spatial density function. The integral over the spatial density function after denoising may be used to obtain an integer value representing the number of molecular substructures.

Note that the number of molecular substructures need not be defined before or during the denoising process. Indeed, the number of molecular substructures may increase or decrease as denoising progresses. After denoising the resulting spatial density function may be discretized by fitting a sum of predefined density distributions. Typically, the predefined density distributions are Gaussians, but other examples are given herein. The centers of the fitted density distributions, e.g., their means, may be taken as positions of the molecular substructures of the molecular structure.

Interestingly, the process allows representing an arbitrary number of molecular substructure positions, which may increase and/or decrease during the denoising.

An effective way to represent a spatial function as a data structure is to represent it as a series of positions of a plurality of points and values of the spatial function. For example, the trained neural network may be configured to modify the positions and/or values of the plurality of points.

A molecular structure generating system and/or a system for training the neural network therefor is an electronic device or system of electronic devices, e.g., one or more computers.

The molecular generation method described herein may be applied in a wide range of practical applications. Such practical applications include: generating molecular structures for exploration, e.g., finding novel and useful molecular structures, e.g., for pharmacological or other chemical applications; inferring the configuration of a molecule from chemical measurements; and debugging, testing, and/or benchmarking chemical software.

An aspect is a method for generation and/or training. An embodiment of the method may be implemented on a computer as a computer-implemented method, in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer-readable medium for performing an embodiment of the method when said program product is executed on a computer. For example, a computer-readable medium may be a computer-readable storage medium.

In an embodiment, the computer program comprises computer program code adapted to perform all or part of the steps of an embodiment of the method when the computer program is run on a computer. Preferably, the computer program is embodied on a computer-readable medium.

### Brief Description

Further details, aspects, and embodiments will be described, by way of example only, with reference to the drawings. Elements in the figures are illustrated for simplicity and clarity and have not necessarily been drawn to scale. In the figures, elements which correspond to elements already described may have the same reference numerals. In the drawings,
Figure 1 schematically shows an example of an embodiment of molecular structure system,
Figure 2a schematically shows an example of an embodiment of a molecular structure generator system,
Figure 2b schematically shows an example of an embodiment of a denoising training system,
Figure 3a schematically shows an example of an embodiment of generation of a molecular structure,
Figure 3b schematically shows an example of an embodiment of an initial noisy spatial density function,
Figure 3c schematically shows an example of an embodiment of intermediate molecular structure,
Figure 3d schematically shows an example of an embodiment of a molecular structure generated before discretization,
Figure 4 schematically shows an example of an embodiment of a continuous graph representing a molecular structure,
Figure 5a schematically shows an example of an embodiment of a continuous graph representing a molecular structure,
Figures 5b and 5c schematically show an example of an embodiment of a disturbed continuous graph,
Figure 6 schematically shows an example of an embodiment of a method for generating molecular structures,
Figure 7a schematically shows a computer-readable medium having a writable part comprising a computer program according to an embodiment,
Figure 7b schematically shows a representation of a processor system according to an embodiment.

### Reference signs list

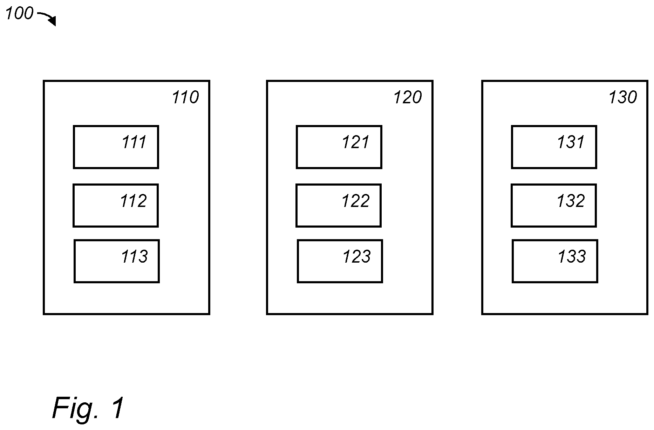

The following list of references and abbreviations corresponds to Figures 1-5c, and is provided for facilitating the interpretation of the drawings and shall not be construed as limiting the claims.
100 a molecular structure system
110 molecular structure generator computer
120 denoising training computer
130 continuous graph inference computer
111, 121, 131 a processor system
112, 122, 132 storage
113, 123, 133 communication interface
200 a molecular structure generator system
210 initial spatial function generator
211 initial spatial function
220 denoising network
230 integrator
231 molecular substructure number
240 fitting unit
241 first molecular structure
250 connectivity inferring unit
251 second molecular structure
270 molecular structure training data
280 density function conversion unit
281 density function representation
290 diffusor
291-293 diffusion training data
1000, 1001 a computer-readable medium
1010 a writable part
1020 a computer program
1110 integrated circuit(s)
1120 a processing unit
1122 a memory
1124 a dedicated integrated circuit
1126 a communication element
1130 an interconnect
1140 a processor system

### Detailed Description

While the presently disclosed subject matter is susceptible to embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the subject matter that is presently disclosed is not limited to the embodiments only but also includes every other combination of features described herein or recited in mutually different dependent claims.

Some embodiments are directed to generating molecular structures, which may include progressively denoising a spatial density function by iteratively applying a trained neural network. The neural network is trained to transform noisy spatial density functions into structured spatial density functions based on training data comprising molecular structures represented as spatial density functions. After denoising, a sum of predefined density distributions is fitted to the denoising result to obtain the molecular structure.

Figure 1 schematically shows an example of an embodiment of a molecular structure generator computer 110, an embodiment of a denoising training computer 120, and an embodiment of a continuous graph inference computer 130.

Shown is a molecular structure generator computer 110, denoising training computer 120, and continuous graph inference computer 130. Molecular structure generator computer 110 and denoising training computer 120 may be comprised in a molecular structure system 100. Optionally, molecular structure system 100 may also comprise continuous graph inference computer 130.

Molecular structure generator computer 110 is configured to generate molecular structures. A generated molecular structure comprises at least a plurality of molecular substructure positions, e.g., atom positions, amino acid positions, chemical group positions, etc. A generated molecular structure may comprise additional information, e.g., bonding information between the molecular substructure positions, atom types, chemical and other information, as further explained herein. Generating molecular structures is useful for a variety of applications, ranging from technical debugging of other software applications to compound discovery.

Molecular structure generator computer 110 uses a neural network trained to progressively denoise a spatial density function. Denoising training computer 120 may be used to train that neural network.

Intermediate data structures of molecular structure generator computer 110 use a continuous representation of a graph structure. In the output of molecular structure generator computer 110, this data structure may be discretized and/or transformed into traditional representations of molecular structures, e.g., a graph or a sequence of substructure positions, e.g., atom positions. Computations, e.g., estimating chemical properties of the generated molecular structure, may then be performed by using traditional graph computation algorithms, in particular graph neural networks. Interestingly, the inventors found that neural network computations may be performed directly on continuous graph representations.

Molecular structure generator computer 110 may comprise a processor system 111, a storage 112, and a communication interface 113. Denoising training computer 120 may comprise a processor system 121, a storage 122, and a communication interface 123. Continuous graph inference computer 130 may comprise a processor system 131, a storage 132, and a communication interface 133.

In the various embodiments of communication interfaces 113, 123, and/or 133, the communication interfaces may be selected from various alternatives. For example, the interface may be a network interface to a local or wide area network, e.g., the Internet, a storage interface to an internal or external data storage, an application interface (API), etc.

Molecular structure generator computer 110, denoising training computer 120, and continuous graph inference computer 130 are represented here as single devices, though any of them could just as well be implemented as systems, e.g., a geographically distributed system, e.g., a cloud computing system, e.g., a system comprising multiple computers. Further, any one of molecular structure generator computer 110, denoising training computer 120, and continuous graph inference computer 130 could be implemented as a process running on a computer, e.g., a cloud computing system.

Storage 112, 122, and 132 may be, e.g., electronic storage, magnetic storage, etc. The storage may comprise local storage, e.g., a local hard drive or electronic memory. Storage 112, 122, and 132 may comprise non-local storage, e.g., cloud storage. In the latter case, storage 112, 122, and 132 may comprise a storage interface to the non-local storage. Storage may comprise multiple discrete sub-storages together making up storage 112, 122, and 132.

Storage 112, 122, and/or 132 may be non-transitory storage. For example, storage 112, 122, and/or 132 may store data in the presence of power, such as a volatile memory device, e.g., a Random Access Memory (RAM). For example, storage 112, 122, and/or 132 may store data in the presence of power as well as outside the presence of power, such as a non-volatile memory device, e.g., Flash memory. Storage may comprise a volatile writable part, say a RAM, and/or a non-volatile writable part, e.g., Flash. Storage may comprise a non-volatile non-writable part, e.g., ROM, e.g., storing part of the software.

Devices 110**,** 120, and/or 130 may communicate internally, with each other, with other devices, external storage, input devices, output devices, and/or one or more sensors over a computer network. The computer network may be an internet, an intranet, a LAN, a WLAN, a WAN, etc. The computer network may be the Internet. Devices 110, 120, and/or 130 may comprise a connection interface which is arranged to communicate within molecular structure system 100 or outside of molecular structure system 100 as needed. For example, the connection interface may comprise a connector, e.g., a wired connector, e.g., an Ethernet connector, an optical connector, etc., or a wireless connector, e.g., an antenna, e.g., a Wi-Fi, 4G, or 5G antenna.

Communication interface 113 may be used to send or receive digital data, e.g., data representing the initial noisy spatial function, intermediate denoised spatial density functions, and computed integrals for further processing. Communication interface 123 may be used to send or receive digital data, e.g., training data sets and neural network parameter updates for refining the denoising process. Communication interface 133 may be used to send or receive digital data, e.g., continuous graph representations, neural networks trained to compute on continuous graph representations.

Molecular structure generator computer 110, denoising training computer 120, and continuous graph inference computer 130 may have a user interface, which may include well-known elements such as one or more buttons, a keyboard, a display, a touch screen, etc. The user interface may be arranged for accommodating user interaction for initiating molecular structure generation operations, training, and/or continuous graph computations.

The execution of devices 110, 120, and/or 130 may be implemented in a processor system. Devices 110, 120, and/or 130 may comprise functional units to implement aspects of embodiments. The functional units may be part of the processor system. For example, functional units shown herein may be wholly or partially implemented in computer instructions stored in a storage of the device and executable by the processor system.

The processor system may comprise one or more processor circuits, e.g., microprocessors, CPUs, GPUs, etc. Devices 110, 120, and/or 130 may comprise multiple processors. A processor circuit may be implemented in a distributed fashion, e.g., as multiple sub-processor circuits. For example, devices 110, 120, and/or 130 may use cloud computing.

Typically, molecular structure generator computer 110, denoising training computer 120, and continuous graph inference computer 130 each comprise one or more microprocessors which execute appropriate software stored at the device; for example, that software may have been downloaded and/or stored in a corresponding memory, e.g., a volatile memory such as RAM or a non-volatile memory such as Flash.

Instead of using software to implement a function, devices 110, 120, and/or 130 may, in whole or in part, be implemented in programmable logic, e.g., as a field-programmable gate array (FPGA). The devices may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, molecular structure generator computer 110, denoising training computer 120, and continuous graph inference computer 130 may comprise circuits, e.g., for cryptographic processing and/or arithmetic processing.

In hybrid embodiments, functional units are implemented partially in hardware, e.g., as coprocessors, e.g., arithmetic coprocessors, and partially in software stored and executed on the device.

Embodiments use continuous graph representations, a novel approach that extends the concept of traditional geometric graphs. The mathematical formalism allows for both inference and generative tasks to be performed on these continuous graph representations.

By treating graphs as continuous objects, this method eliminates the requirement to predefine the number of nodes in a graph. Furthermore, this approach is resolution-adaptable, enabling the use of different resolutions for the continuous graph representations during both training and inference phases.

The continuous graph representations can be used for inference tasks on geometric graphs. For example, they can predict the energy of a molecule or classify meshes. The method also allows for generative modeling of both graphs and meshes.

Continuous graph representations can be used for generating graphs and meshes. Given a dataset, it can be used to generate new data either conditionally or unconditionally. In an embodiment, this may be done in a resolution-free way.

The motivating example and current primary use for continuous graph representations are in molecule/drug design. A further use case is in mesh generation for simulation models. For example, molecule configurations may be generated for drug development or other chemical engineering processes.

In a possible molecule design process, existing 3D configurations of molecules are used as training data, and candidates for plausible 3D configurations of molecules are generated. For example, molecules may be tested experimentally, e.g., in a wet lab.

A new 3D configuration of a molecule can be used as input geometry for a three-dimensional partial differential equation (PDE) simulation, where its spatial coordinates define the simulation domain and boundary conditions. This simulation may comprise numerically solving a three-dimensional partial differential equation to compute properties of the molecule, such as electron density or electrostatic potential.

For example, an embodiment may be configured to generate 3D molecular structures, e.g., molecules. These may be novel molecular structures, which do not need manual domain expert generation. Generated molecular structures may be evaluated according to various criteria, as further set out herein. Selected molecular structures may be synthesized.

In an embodiment, discrete graph structures are generalized to continuous field representations, which can represent both discrete graphs and intermediate constructs, e.g., between two traditional discrete graphs. Properties such as node density, edge density, and other features may be represented as continuous fields. Interestingly, these continuous field representations, also referred to as excitation fields, can be used to generate new graphs with an arbitrary number of nodes; in particular without predetermining a set number of nodes. In a motivating example, the graphs represent molecular structures. For example, a graph may represent a molecule and the graph nodes may represent atoms.

Graphs are represented as continuous objects in space. Consider a geometric graph where each node is embedded in a n-dimensional space, and has a position vector $r ∈ {ℝ}^{n}$ assigned to it. The key element is a function *ρ*ₙ(r) which represents node density at a location r. For a graph with N nodes, this density should integrate to *N.*

Optionally, additional functions may be used.

h(r): A feature field evaluated at the point r. The feature field can be considered as a generalization of a feature-vector, as each location in space now has a vector value assigned to it.

*ρₑ*(**r**₁*,* **r**₂): Represents the edge density existing between the locations **r₁** and **r₂**. For a graph with *E* edges, this density should integrate to *E*. This function is optional. In fact in many embodiments, the function is omitted. In such a case the location of edges is implicit from the location of the graph nodes, e.g., atoms.

We shall interchangeably refer to this continuous representation of graphs as a field. Thus, when we refer to a graph field, we are referring to the set of node densities, and optionally feature field(s), and/or edge densities.

Conventional discrete graphs can be expressed within the framework of continuous graphs. Consider a graph comprising of a set of nodes *nᵢ* and edges *eᵢⱼ*, where *eᵢⱼ* = 1 indicates that nodes *i* and *j* are connected. Each node *i* is positioned at **r***ᵢ* and possesses a feature vector **h***ᵢ*. In this context, the field functions can be represented as follows: ${ρ}_{n} \left(r\right) = {\sum }_{i}^{N} δ \left(r-{r}_{i}\right)$, e.g., it may be a sum of Dirac delta functions. This is because the nodes have deterministic positions. ${ρ}_{e} \left({r}_{1}, {r}_{2}\right) = {\sum }_{i = 1}^{N} {\sum }_{j = 1}^{N} δ \left({r}_{1}-{r}_{i}\right) δ \left({r}_{2}-{r}_{j}\right) {e}_{ij}$: This can be understood as follows: an edge can only exist between two points if both of them are nodes (indicated by the two delta functions), and if those two points share an edge in the original graph, denoted by e*ᵢⱼ.*$h \left(r\right) = {\sum }_{i}^{N} δ \left(r-{r}_{i}\right) {h}_{i}$: This function may be a weighted sum of Dirac delta functions, where each node's feature vector **h***ᵢ* is associated with its respective position **r***ᵢ*.

It is important to highlight that the densities mentioned, e.g., the node density, are not normalized. This fact is used to advantage when generating graphs, e.g., a molecular structure, that do not have a predefined number of nodes-it is not necessary to define before generating how many nodes should be generated. Rather the number of nodes can be changed organically during generation. This leads to significantly more natural structures that better bit the empirical distribution of the training data.

In an embodiment, the node density has a total density mass of *N* representing the number of nodes. If an edge density is used, it has a mass of *E* indicating the total number of edges. To constrain the vast space of potential functions, some reasonable assumptions regarding the edge density function *ρₑ* (**r**₁, **r**₂) may be introduced. Intuitively, for two random points in space, the likelihood of an edge existing between them is low unless nodes are present at those specific locations. Therefore, the first important property of this function is that it should assign higher values to pairs of locations where the node existence densities are higher. This relationship naturally emerges in the limiting case of a regular fully-connected graph (*eᵢⱼ* = 1 for every pair of nodes), as we can observe that in such cases, the edge and node densities are related: ${ρ}_{e} \left({r}_{1}, {r}_{2}\right) = {\sum }_{i = 1}^{N} {\sum }_{j = 1}^{N} δ \left({r}_{1}-{r}_{i}\right) δ \left({r}_{2}-{r}_{j}\right) = {ρ}_{n} \left({r}_{1}\right) {ρ}_{n} \left({r}_{2}\right)$

Motivated by this observation, the edge function may be modelled as: ${ρ}_{e} \left({r}_{1}, {r}_{2}\right) = {ρ}_{n} \left({r}_{1}\right) {ρ}_{n} \left({r}_{2}\right) f \left({r}_{1}, {r}_{2}\right) ,$ where *f*(**r**₁, **r**₂) is a function.

In many scenarios, explicit edge indices may not be present. For example, in molecular structures, edges may be implicit. For example, e.g., as observed in molecular structures edges, e.g., edges representing an atom bonds, may be inferred, e.g., based on proximity.

For example, a quick approximation, which may be used for graph computations such as further disclosed herein a function may be used that scales edge density interaction based on the distance between nodes. For example, such function could be: $f \left({r}_{1}, {r}_{2}\right) = {e}^{α {\left({r}_{1}-{r}_{2}\right)}^{2}}$. However, in general, we will not necessarily assume this specific form of the function *f*(**r**₁, **r**₂). In particular, in many generation embodiments, no explicit modelling of edges is done or needed, as edges may be inferred using traditional computational chemistry software.

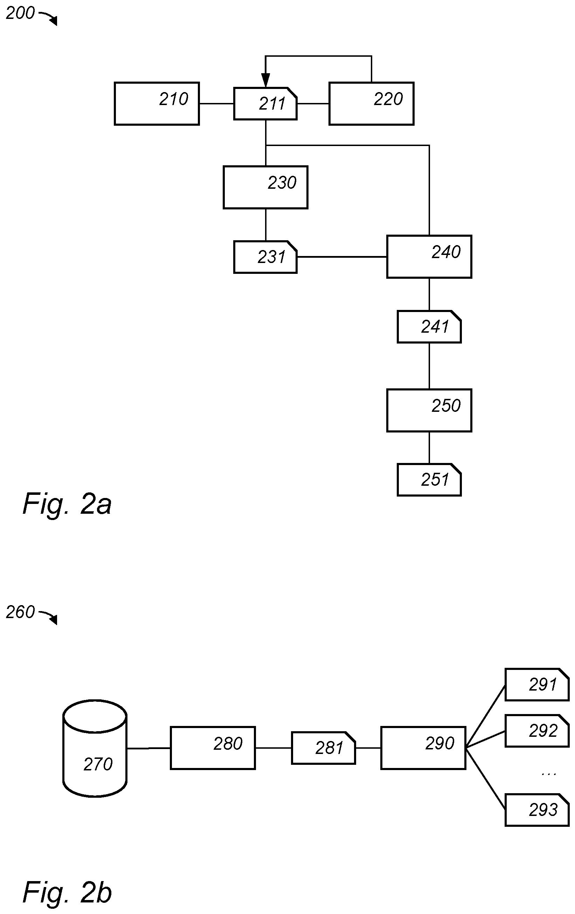

Figure 2a schematically shows an example of an embodiment of a molecular structure generator system 200. System 200 uses continuous graph representation, e.g., such as described above, to generate realistic molecular structures; for example, the molecular structures may be obtained as drawn from an empirical distribution as defined by a training set.

Embodiments may be applied to various types of molecular structures. A molecular structure comprises at least a plurality of molecular substructure positions. A molecular structure may further comprise bonding information between the molecular substructures and/or other information regarding the molecular structure.

In our motivating example, which was found to work effectively in a prototype, the molecular structures may be molecules. In this case, the molecular substructures may comprise atoms. Accordingly, in an embodiment, molecular structures are generated comprising at least a plurality of atom positions of the atoms in the molecule.

Other molecular structures are possible. In an embodiment, the molecular structure comprises a protein, wherein the molecular substructures are amino acids. In an embodiment, the molecular structures comprise chemicals, wherein individual chemical groups (such as hydroxyl, methyl, or carboxyl groups) act as molecular substructures. Embodiment described for molecules and atoms may be adapted to operate on these other molecular structures and substructures.

In an embodiment, a molecular structure, e.g., a molecule, is represented as a density function. In particular, the positions of the molecular substructures are represented in the density function. For example, a molecular structure of a physical molecular structure may be represented as a density function as follows. First, the molecular substructures of the physical molecular structure are represented in a spatial domain, e.g., a part of 2D or 3D space, preferably with positions corresponding to the physical positions of the physical molecular substructures. Typically, 3D space is used for the spatial domain. Using 3D space has the advantage that modelled positions naturally correspond to physical positions. However, the generation process may be used for any positive dimension.

The molecular substructures can be represented as a sum of density functions, wherein the density functions correspond to the molecular substructures, and the probability density function is centered on the position of the corresponding molecular substructure, e.g., an atom.

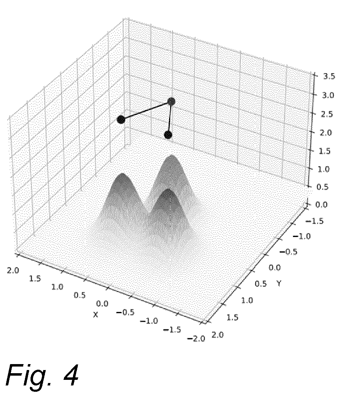

Figure 4 schematically shows an example of an embodiment of a continuous graph representing a molecular structure. In this case, a water molecule has been represented in 2D space, which in turn is represented as a sum of density functions. The figure shows a 3D representation of the resulting density function for the water molecule. The atom positions and bonds are indicated in the figure as a traditional graph, superimposed above the density function. Note that the integral over the density function is 3.

In an embodiment, the molecular structures are molecules, and the molecular substructures are atoms. A spatial density function represents the position of the atoms. In an embodiment, the molecular structures are proteins. Distinct peaks of the spatial density function correspond to individual amino acid centers.

Structures that are in the process of generation are likewise spatial functions, e.g., functions from the spatial domain, e.g., a part of 2D or 3D space, to the real numbers. However, during generation, the function does not necessarily have to be a density function; that is, the function may be allowed to map to negative numbers. Note that a density function is a function from the spatial domain to non-negative numbers.

In an embodiment, the spatial functions are spatial density functions throughout generation, though, as said, this is not necessary.

There are various ways to represent a spatial function and/or spatial density function in a computer. In a first approach, a regular grid of voxels is applied to the spatial domain. In this case, the spatial function is sampled at the positions of the regular grid, e.g., a Cartesian grid, a honeycomb grid, etc. During generation, the values at the regular grid are refined to converge on a molecular structure. In this case, the number of values needed to represent a spatial function grows with the third power of the resolution. When a larger resolution is used, e.g., in order to reliably represent larger molecules, the amount of sampled values grows quickly. As a result, denoising operations operate on more data, and thus become slower. Furthermore, the neural network used for denoising will require more parameters, making it slower and requiring more training data.

The inventors found an alternative way to represent a spatial function. Here, a spatial function is represented as a series of tuples *(xᵢ, pᵢ),* wherein the xᵢ are vectors in the spatial domain, and *pᵢ* is a number, e.g., a real number. For a spatial density function the *pᵢ* are non-negative or preferably, positive numbers. It was found that this representation presents many advantages. First of all, the number of samples does not grow with the size of the spatial domain. Although a higher resolution of the spatial function will use more samples, the number of samples needed turns out to grow much smaller. It was found that *O*(*n*) samples, wherein n represents the maximal number of molecular substructures, appear to work well. For example, in an embodiment, for an upper bound of molecular substructures of n, 100n or fewer samples are used. In an embodiment, 10n or fewer samples are used. For example, between 10n and 100n samples.

Even if some tolerance is allowed for increased complexity that may arise with an increasing number of molecular substructures, e.g., *O*(*n* log *n*) samples may be used, e.g., 100 n log n or fewer samples, this still this grows significantly slower than the third power. For example, between nlogn and 100nlogn samples, e.g., between 10*n* log *n* and 100*n* log *n* samples. Note, that in voxel representation increasing resolution, that is an increasing number of voxels, is needed for increasingly large molecules. In sample representation, increasing accuracy may be obtained by enlarging the spatial domain, or increasing the accuracy of the representation of the positions, e.g., an increased number of digits.

### Generation: Initial Noisy Spatial Density Function

Initial spatial function generator 210 is configured for generating an initial noisy spatial function 211 and representing the spatial function in a data structure. The data structure may be according to a grid or, preferably, according to a list of sampled values.

Returning to Figure 2a, system 200 comprises an initial spatial function generator 210. For example, system 200 may generate a sequence of *(xᵢ, pᵢ),* wherein the *xᵢ* are randomly selected from the spatial domain, and the *pᵢ* are randomly selected from a suitable interval. The values *pᵢ* and xᵢ are treated as a tuple, because the value of *pᵢ* is determined by the value of the density of the field at the location *xᵢ*, so the locations and values are entangled.

A suitable interval may be derived from the training process, as the interval for the values *pᵢ* should correspond to the maximally disturbed values in the disturbed training data (see below). The range for *pᵢ* may be constraint in that the field integrates to a particular number. Such a constraint will influence though typically not determine the number of nodes in a graph. This may be used to coax the search into a particular direction. For example, a user may set the particular number, after which the tuples are selected so that an integral over the field represented by the tuples equals or at least approximates the particular number.

As an example, the values *pᵢ* may be selected from -1 to 4, though larger or smaller intervals are possible. Typically, as more denoising iterations are used, a larger interval for the values *pᵢ* is used. The vector values of *xᵢ* are less critical. For example, they may be chosen to correspond to values observed in training. For example, the spatial domain may be the cubed unit interval. In an embodiment, the cubed interval [-4, 6] is used for the points *xᵢ*. As resolution depends on the number of samples and the accuracy of the real number representation, but not on the size of the spatial domain, this is less critical. Various distributions for *xᵢ* and *pᵢ* are possible. For example, the distribution may be uniform. The distribution may be Gaussian, preferably with a large variation.

### Generation: Progressive Denoising

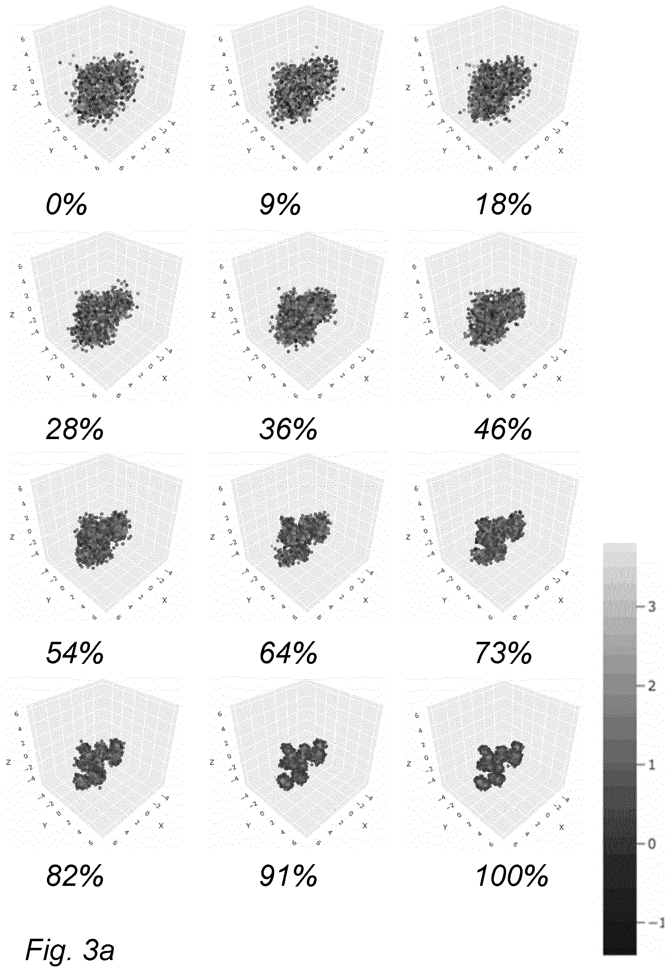

The noisy spatial function 211 is progressively denoised by iteratively applying a trained neural network 220. Various numbers of iterations can be used. Interestingly, even with comparatively low numbers of iterations, good results can be obtained; probably due to the fitting process further improving the result. For example, in an embodiment, at least 10, at least 100, or at least 1000 iterations are used. In the worked example shown in Figures 3a-3d, 150 iterations were used. In an embodiment, a different number of iterations may be used, either a higher or lower number. In a more efficient embodiment, fewer than 150 iterations are used. In an embodiment, at least 10 iterations are used, e.g., between 10 and 150 iterations.

The neural network is trained to transform noisy density functions eventually into structured spatial density functions based on training data comprising molecular structures represented as spatial density functions. A possible training scheme for neural network 220 is presented below.

Interestingly, the number of molecular substructures, e.g., atoms, in the final spatial density function corresponds to an integral over the density function. Interestingly, the integral over the spatial function can vary during the denoising process. This means that the number of molecular substructures, e.g., atoms, that are represented is not fixed but can vary. As a practical limit, a higher number of atoms needs more resolution to be adequately represented, but this can be accounted for by a linear increase in the number of samples.

Interestingly, by repeatedly applying a denoising network, the initial random configuration converges on a configuration drawn from the empirical distribution of the training data.

The neural network may work on point sets. For example, in an embodiment, the neural network receives as input a point cloud rather than a fixed grid. In an embodiment, the neural network comprises one or more convolution operators defined to operate on a point set. For each input point *(xᵢ, pᵢ),* a local neighborhood is defined-for example, the k-nearest neighbors or all points within a fixed radius. Then, a weighted sum of the densities *pⱼ* of the neighboring points x*ⱼ* is computed. However, the kernel weights are not fixed numbers in a grid; but it given by a function *k*(*xᵢ,xⱼ*) that depends on the relative spatial positions *xᵢ* and *xⱼ* and is defined by the network.

Mathematically, for each point *xᵢ*, the convolution-like operation could be defined as: *Σj*_{∈*N*(*xi*})*k*(*xᵢ,xⱼ*) *· pⱼ*. Here, *N(xᵢ)* denotes the neighborhood of *xᵢ*, and *k* is defined by a neural network. This operation aggregates the local information in a way that is invariant to the ordering of the points. The results of the point cloud convolutions are input to further layers, e.g., further convolutions, and/or pooling operations.

In an embodiment, the point cloud technique disclosed in "On the Utility of Equivariance and Symmetry Breaking in Deep Learning Architectures on Point Clouds" by Vadgama et al. is used.

In an embodiment, the spatial functions are presented as samples taken along a regular grid. Although this is slower, it does allow the use of conventional convolutions.

### Feature Fields

Denoising spatial functions, e.g., to obtain spatial density functions that represent molecular structures is surprisingly effective. The inventors found two complementary ways to include more information during the generation: conditional generation and feature fields. Feature fields are particularly effective, as they provide more information after generation is complete, e.g., information beyond the positions of molecular substructures. Moreover, feature fields allow the incorporation of more chemical data during training. This leads to improved performance of the denoising neural network and thus more accurate results.

A feature field is a spatial function from the same spatial domain. The feature field may have values of categorical type, e.g., atom type, and numerical type, e.g., charge. For categorical data, the feature field may map to vectors, in particular, one-hot vectors. For example, a one-hot vector may encode an atom type, using a different vector element for each atom type. This allows the feature field to vary continuously during generation while converging to a one-hot vector.

Like the spatial function, a feature field is also progressively denoised by iteratively applying a trained neural network. Preferably, this uses the same neural network as the one denoising node density, to take advantage of the additional structural information in the feature fields.

Feature fields may be used to represent a range of information, e.g., one or more of the following: atom type, charge, electronegativity, partial atomic charge, hybridization state, van der Waals radius, bonding preferences, and polarizability.

For example, in the case of the water molecule of Figure 4, a feature field may encode the atom types. This may be regarded as a mapping from *R*² (coordinates) to *R*² (two atom types). However, there is also the node density, which is a scalar-valued field. Note that typically, feature fields map from *R*³ if a 3D spatial domain is used. In the example of Figure 4, the feature field uses a one-hot vector of dimension two, however in other molecular tasks, there may be more than two dimensions, e.g., because there are more atom types.

Interestingly, it was found that no regularization is needed to keep the density function and feature fields consistent. The model learns that there are correlations between the two. It turned out not to be necessary to impose conditions on the density function and feature field(s). In an embodiment, the neural network takes as input both a spatial function, possibly specified as a list of pairs of a vector and a value, e.g., a density, and a feature field. The feature field may also be represented as a list of pairs and a value or vector of values. The value or values may be a density. The neural network is configured to produce as output both a denoised spatial field corresponding to spatial density and a denoised feature field.

Below a number of embodiments are described using more mathematical language. In an embodiment, spatial functions, e.g., excitation fields, are generated that can later be converted to discrete graphs. During training, graphs are converted to fields, as described herein. This creates the ability to calculate losses during training directly in a continuous space, rather than having to convert fields back to graphs, which would typically require some non-differentiable operation. On a high-level, the sampling process can be summarized as:
Initiate the process with a noisy node density *ρ*(**r**), and optionally noisy feature fields **h**(**r**).

Denoise both the node densities and optionally the feature fields.

As a result, a node density *ρ*(**r**) is obtained, and optionally also a feature field **h**(**r**).

To avoid the representation of these fields as images and/or voxels, we generative models, e.g., denoising neural networks, may be used that operate directly on a sampled field representations, e.g., wherein the final representation is of the form of a sequence of pairs (*xᵢ, ρ*(*xᵢ*)) for the node density, and a sequence of pairs (*xᵢ, h*(*xᵢ*)) for the feature field. Preferably, *ρ*(*xᵢ*) is a non-negative or even positive function. Preferably, the integral *ρ* corresponds to the number of molecular substructures, e.g., atoms. This condition may also be imposed on *h*(*xᵢ*)*,* e.g, non-negative or positive values or vectors with non-negative or positive values. However for the feature field this is not necessary, especially if the modeled values may be negative. It is noted that for the intermediate spatial functions during the denoising process non-negativity constraints may be relaxed. In particular, negative values may be included in the initial spatial function to start the denoising process.

Formally, Denoising Diffusion Probabilistic Models (DDPMs) are a type of latent variable model. They aim to learn a parametric data distribution *p*_{θ} (**x**₀) from an empirical distribution of finite samples *q*(**x**₀). This is achieved by reversing the forward diffusion process that generates latent variables **x**_{1:*T*} by gradually adding noise to the data, typically Gaussian noise. We develop **x**₀ ~ *q*(**x**₀) over *T* timesteps as follows: $q \left(\left.{x}_{t}\right|{x}_{t - 1}\right) : = N \left({x}_{t};\sqrt{{\overline{α}}_{t}}{x}_{0},\left(1-{\overline{α}}_{t}\right)I\right)$

Here, *α̅ₜ* is the cumulative product of fixed variances that are obtained from a noise scheduler. For our purposes, we may use a simple linear scheduler.

By reparametrizing the previous equation as ${x}_{t} = \sqrt{{\overline{α}}_{t}} {x}_{0} + \sqrt{1 - {\overline{α}}_{t}} ϵ$*,* it can be shown that the simplified loss can be used for training: ${L}_{θ} = {E}_{t ∼ \left[0, T\right] , {x}_{0} ∼ q \left({x}_{0}\right) , ϵ ∼ N \left(0, I\right)} \left[{\left‖ϵ-{ϵ}_{θ}\left(\sqrt{{\overline{α}}_{t}}{x}_{0}+\sqrt{1 - {\overline{α}}_{t}}ϵ,t\right)\right‖}^{2}\right] ,$ where *ε_{θ}* is the denoising model parametrized by *θ*. The process is well-defined when the data **x**₀ is a vector from an *N*-dimensional vector space ${ℝ}^{N}$. However, in our setup, we are interested in generating densities *ρ*(**r**), which are continuous functions. The generalization to this case is not straightforward. Nonetheless, a rigorous mathematical formulation of such a problem exists.

We propose an improved version of these methods, serving as a natural generalization, wherein a Gaussian process (GP) is used instead of independent Gaussian noise. A Gaussian process may be regarded as a random function with spatial correlations defined by a kernel. Formally, we can express the noisy density *ρ*(**r**) at time step *t* as: ${ρ}_{t} \left(r\right) = \sqrt{{\overline{α}}_{t}} {ρ}_{0} \left(r\right) + \sqrt{1 - {\overline{α}}_{t}} g \left(r\right) ,$ where *g*(**r**) is a Gaussian process evaluated at the coordinate r. Consequently, the optimization target may be modified to: ${L}_{θ} = {E}_{t ∼ \left[0, T\right] , {ρ}_{0} \left(r\right) ∼ q \left({ρ}_{0}\left(r\right)\right) , g ∼ {GP}_{Θ} , {r}_{i} ∼ X} \left[{\left‖g\left({r}_{i}\right)-{ϵ}_{θ}\left(\sqrt{{\overline{α}}_{t}}{ρ}_{0}\left({r}_{i}\right)+\sqrt{1 - {\overline{α}}_{t}}g\left({r}_{i}\right),t\right)\right‖}^{2}\right] ,$ where additionally the expectation is taken over the field evaluations at a finite number of potentially non-uniformly sampled locations **r***ᵢ* on a domain 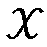 , and we sample a random function *g*(**r**) from a GP parameterized by *Θ*. Note that for generating both densities *ρ*(**r**) and feature fields **h**(**r**), an embodiment follows the same procedure as described above. Interestingly, this can be implemented by concatenating them and treating the result as a single vector field.

Another approach, which may be used together or instead of feature fields is conditional generation. In an embodiment the generation of the molecular structure is conditioned on one or more conditioning parameters. For example, the one or more conditioning parameters may specify desired molecular properties or structural constraints. For example, the one or more conditioning parameters may be obtained from a user, read from a file, or the like.

The conditioning parameters can include both quantitative molecular descriptors and qualitative structural constraints. For instance, these parameters may represent continuous values such as molecular weight, logP, polar surface area, or the number of hydrogen bond donors/acceptors, as well as categorical constraints like the presence of specific functional groups, ring sizes, or connectivity patterns.

Conditioning parameters may comprise, e.g., a class label, or a text description, etc. In an embodiment, conditioning parameters are preprocessed, e.g., continuous values may be normalized, while categorical attributes may be encoded, e.g., via one-hot encoding or learned embeddings.

Optionally, the conditioning parameters may be encoded into a vector, possibly using an embedding layer or an encoder network. This vector serves as a compact representation of the auxiliary data.

To introduce conditioning into this framework, the network architecture and/or the training procedure may be adapted in various ways so that the denoising process is informed by the conditioning parameters. The neural network is trained to transform noisy spatial density functions into structured spatial density functions conditioned by the one or more conditioning parameters.

Note that conditioning parameters are used both during generation and during training. During training, the network learns to generate molecular structures that satisfy the specified properties and constraints by minimizing the prediction error between the denoised outputs and the target structures.

Integrating the conditioning parameters into the denoising network can be achieved through various methods. For example, the input of the denoising network (e.g., the noisy spatial function) may be extended with one or more conditioning parameters. For example, the conditioning vector may be appended to the noise input or intermediate feature maps. The one or more conditioning parameters may be provided to the trained neural network when applied to the spatial function.

This is preferably done at each denoising step. Other approaches include incorporating conditional normalization layers into the network (e.g., FiLM layers) that use the conditioning vector to modulate activations, and/or incorporating cross-attention layers that allow the network to selectively focus on different parts of the conditioning vector during the denoising process.

### Discretizing

Returning to Figure 2a ,at some point, the iterative denoising by repeatedly applying a denoising neural network is terminated. Termination may be indicated in various ways. For example, the system may be configured for a fixed number of iterations. The fixed number may be the same as or proportional to the number of disturbing steps used during training. Another approach is to use a convergence criterion. The denoising process may be terminated when the difference between successive denoised outputs becomes minimal, indicating convergence. For example, a norm, say L2 or L1 norm, of the differences may be below a threshold to decide that the changes are minimal.

Once denoising has terminated, the continuous graph representation may be converted to a discrete graph. In this case, the discrete graph may not have explicit edges. The edges may be left implicit, as they are suggested by molecular substructure positions and possibly feature fields. For example, molecular substructure positions may be atom positions. For example, a feature field may be atom type.

In an embodiment, an integral of the spatial function produced when the last denoising step is computed. As the targets during training are spatial density functions, it is very likely that the final spatial function is a spatial density function.

An embodiment may explicitly check if the spatial function is not a spatial density function, and if so, an error may be reported. However, assuming the integral over the spatial function is positive, the discretization may still proceed.

Based on the integration of the spatial function, an integer number is derived. Typically, this number is a rounding of the integration result, e.g., rounding to the nearest integer, rounding down to an integer, etc. In a variant, a discretization may be performed for slightly different values, e.g., a rounding of the integration plus a small number, e.g., plus one. This will produce slightly different discretizations.

In an embodiment, system 200 comprises an integrator 230 configured to compute an integral over the last spatial function computed by the denoising neural network. Integrator 230 produces a molecular substructure number 231. If the spatial function is represented as values along a regular grid, then the integral may be approximated by adding the values and multiplying with the size of the voxels. If the spatial function is represented as a series of samples, the integral can also be computed, e.g., using then importance sampling or other Monte Carlo techniques may be used.

Next, a sum of a specified number of predefined density distributions is fitted to the final spatial function. In an embodiment, system 200 comprises a fitting unit 240 configured to produce a first molecular structure 241. First molecular structure 241 may comprise a list of positions of the molecular substructures, e.g., atoms.

For example, assume *f*(*x*) is the final spatial function, and *m* is specified number, that is the integer number based on the integration of the spatial function. The objective may be defined as finding µ₁, ..., *µₘ,* so that ${\sum }_{i = 1}^{m} N \left({μ}_{i}\right)$ fits the function *f*(*x*)*.* Here *N*(*µᵢ*) is a distribution with mean *µᵢ.* Typically, a Gaussian with fixed standard deviation, e.g., unit standard deviation is used. The distribution used is typically the same as the distribution used during training when generating the training data. Other distributions than Gaussian can be used, e.g., a Gamma distribution.

For example, an objective function may quantify the difference between the output of the denoising network and the function to be fitted. When the neural network output is given as a list of pairs *(xᵢ, pᵢ),* then the objective function may, e.g., be defined as: *L*(*µ*₁*, ..., µₙ*) *=* Σ*ᵢ Σₖ*(*N*(*xᵢ*; *µₖ*) - *pᵢ*)²*.* Note that here the values *µ*₁, ..., *µ*ₙ are points in the spatial domain, typically a 3D domain.

An optimizer may be used to optimize the objective function and derive the means. For example, a gradient descent optimizer may be used.

In an embodiment, first an initial estimate of the means is obtained using a clustering algorithm, e.g., using k-means, to get an initial estimate of the means, followed by fitting a weighted mixture of gaussians to further approximate the means better.

Once the distributions have been fitted to the data, the centers of the fitted density distributions are identified as positions of the molecular substructures of the molecular structure. In the example, above the means, e.g., the fitted values of *µ*₁, *..., µₙ* may be regarded as the centers of the fitted density distributions.

Once the means are known a molecular structure has been generated. It is possible to enhance the molecular structure in various ways. For example, connectivity between identified molecular substructure positions may be inferred by applying a computational bond determination method to construct a molecular graph. In an embodiment, system 200 comprises a connectivity inferring unit 250 configured to produce a second molecular structure 251. Second molecular structure 251 may comprise the same information as first molecular structure 241 and in addition bonding information. Alternatively, second molecular structure 251 may be a pure graph representation of the molecule with atoms as nodes, bonds as edges. Second molecular structure 251 may also be in traditional formats, e.g., a connection table (Ctab). Second molecular structure 251 may comprise information describing the structural relationships and properties of a collection of atoms. The atoms may be wholly or partially connected by bonds. Such collections may, for example, describe molecules, molecular fragments, substructures, substituent groups, polymers, alloys, formulations, mixtures, and unconnected atoms. Second molecular structure 251 may be in MDL file format.

Various options are known in the art to do this, ranging from traditional computational methods to machine learning approaches. One approach is detailed in "Prediction of organic homolytic bond dissociation enthalpies at near chemical accuracy with sub-second computational cost" , by Peter C. St. John, et al., included herein by reference; and in "Investigating Molecular Geometry: A Comprehensive Analysis Using VSEPR Theory", by Aejaz Ahmad Khan, included herein by reference.

It is noted that bonding information may be applied in discretized form, e.g., as edges in a graph, wherein the molecular substructures are nodes, or as numerical information. Possibly for each edge between molecular substructure nodes, numerical bonding information may be provided. An example of such numerical information is bond order, which quantifies the strength and multiplicity of a bond between two molecular substructure nodes, or bond dissociation enthalpy, which represents the energy required to break the bond.

Instead of or in addition to providing additional information to the discretized generated molecular structure, bonding information could also be computed for a continuous graph representation, e.g., just before discretizing. In this case, continuous values can be used for the presence or absence of a bond. This has the advantage that continuous graph neural network computations can be used, as described herein.

Another way to provide additional information to the discretized molecular structure, with or without explicit bonding, e.g., with or without explicit edges, is to extract information from feature fields that were computed, e.g., denoised, during the generation of the molecular structure.

For example, after discretizing the spatial function indicating the density of molecular substructures, e.g., atoms, one or more denoised feature fields may be sampled at the identified positions of the molecular substructures to obtain feature values associated with each molecular substructure.

The discretized molecular structure may be saved to a file and/or reported to a user. Additional information, e.g., inferred edges such as bonding information, and/or sampled feature fields may likewise be saved to a file and/or reported to a user. Various applications may be provided with the molecular structure information, whether in discretized or continuous form, and/or with or without additional information.

In an embodiment, the method of the invention is used to reconstruct molecular structures, e.g., molecules, based on chemical information. For example, such an embodiment may be used in a forensic setting to determine which molecules were likely to be present based on chemical information. For example, such an embodiment may be used in a chemical testing and/or research setting, where it is needed to know which molecules were present based on chemical information, e.g., to investigate the source of a leak, to determine the identity of a useful molecule or molecular structure, etc.

For example, chemical data is obtained regarding the molecular structure, typically experimentally. For example, experimental data regarding a present molecule may be collected; however, the molecule in question may be unknown. For example, the experimental data may comprise any one of the following: full or partial NMR spectra, full or partial mass spectra, or full or partial MS fragmentation. The experimental data may be provided as conditioning data during the generation process. Generated molecules are likely to fit the experimental data and correspond with the physical-though possibly unknown-molecule. In an embodiment, multiple molecules are generated, and the top-k most occurring molecules are presented.

A further application relates to the testing of chemical software. Chemical software requires rigorous testing to ensure accurate and reliable performance. Such software includes, for example, algorithms for inferring molecular bonds from atomic positions and computational methods for determining chemical properties such as dipole moments or reaction energies. However, testing such software is challenging due to the limited availability of molecular data in public and proprietary databases. Furthermore, the molecular structures and properties present in these databases are often already used during software development, making them unsuitable for independent validation. As a result, testing efforts may be biased or incomplete.

Using random data to test, debug, or benchmark chemical software is generally ineffective. The vast majority of randomly generated structures do not correspond to chemically meaningful molecules, often leading to early termination due to structural errors or unrealistic bonding configurations. Such an approach does not effectively exercise the full functionality of the software, as most test cases fail prematurely rather than probing complex or edge-case behaviors. Consequently, to test chemical software in a way that enables meaningful stress-testing and validation, there is a need for a large amount of realistic molecular structures.

Generating a plurality of molecular structures according to an embodiment provides a solution by creating realistic and chemically valid test cases. Applying a further chemical software, configured to receive a molecular structure, to the plurality of molecular structures allows systematic evaluation of its behavior. During testing, errors may arise due to unexpected molecular configurations or computational failures. The software may be monitored for outright errors, e.g., crashes of the software, memory leaks, etc. Furthermore, the software's output can be monitored for implausible results, e.g., inconsistencies such as physically implausible bond lengths or unrealistic property calculations. When errors or other problems are detected, the specific molecular structure causing the issue can be identified and flagged, allowing developers to analyze the failure.

Additionally, a plurality of molecular structures can be used to benchmark different chemical software implementations by applying various types of further software and comparing their performance under controlled conditions. By timing computations on the same or similar hardware, discrepancies in efficiency can be detected. Differences in execution time, memory usage, or computational stability across similar molecules may indicate inefficiencies or implementation flaws in a given software. Comparative benchmarking can also reveal variations in computed chemical properties, e.g., cases where one implementation diverges from others.

In an embodiment, conditioning information may be used to ensure the generated molecular structures span a diverse chemical space, e.g., covering a wide range of bonding patterns, functional groups, and/or steric configurations.

A further application relates to the synthesis of molecular structures. Generating a molecular structure, in particular a molecule, enables the exploration of new chemical compounds with potential applications in various fields. Applying a retrosynthesis tool to the molecular structure provides a synthetic route by identifying feasible precursor molecules and reaction steps. By following this synthetic route, the molecule can be synthesized and experimentally validated. Synthesis may involve a human chemist carrying out the required reactions in a laboratory, following established procedures and adjusting conditions as needed, or it can be fully automated using robotic synthesis platforms that execute reactions under software control, e.g., leveraging automated reagent dispensing, reaction monitoring, and purification techniques.

In an embodiment, the synthesized molecule is tested for chemical and/or pharmaceutical properties. In an embodiment, the synthesis outcome is monitored for verification of the retrosynthesis tool's accuracy, identifying cases where predicted pathways are impractical, inefficient, or ineffective.

Figure 3a schematically shows an example of an embodiment of the generation of a molecular structure.

Figure 3a shows various intermediate points during generation of a molecule. A percentage below each figure indicates at which point during generation the snapshot was taken.

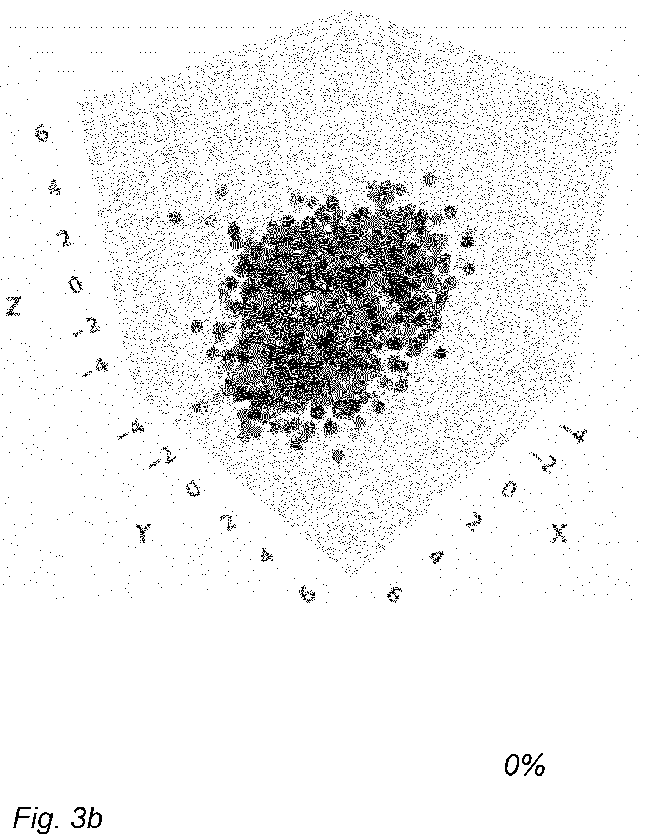

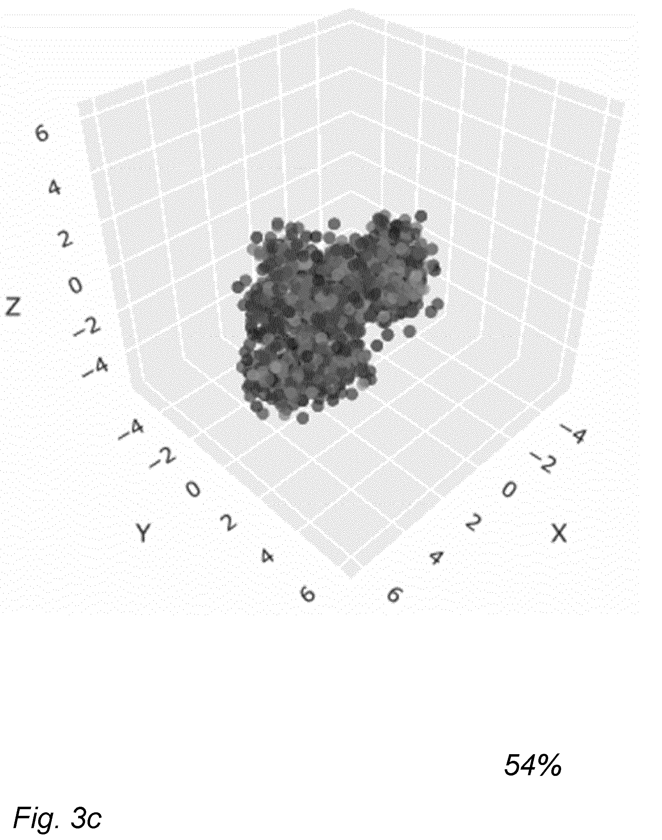

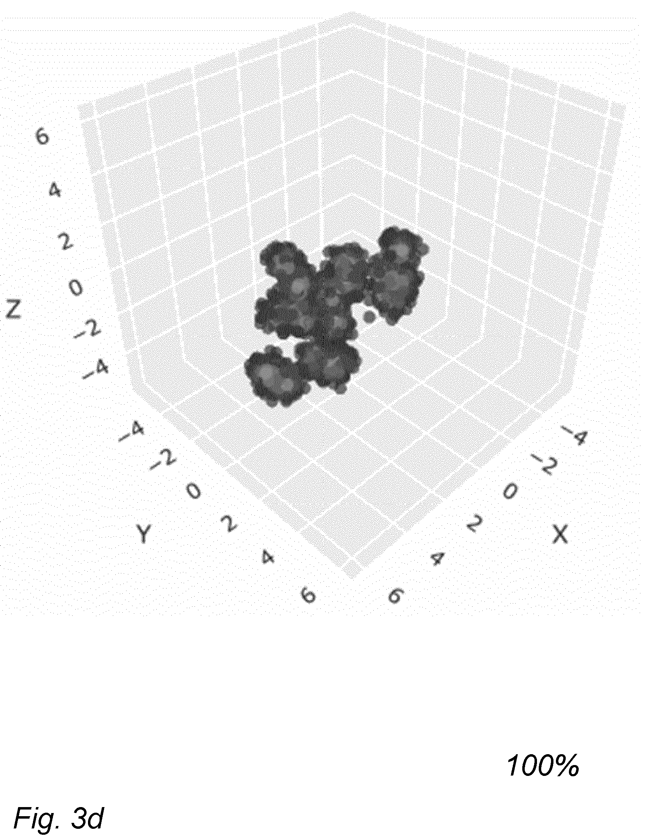

In each of the images, the spatial function is represented as a series of positions of a plurality of points and values of the spatial function. The plurality of points are chosen in a 3D spatial domain. The function is visualized in Figure 3 by plotting the 3D positions of each of the points and indicating the value of the spatial function with a grayscale.

The image at 0% denotes the initial sampling of points before the denoising starts. Note that at this point it is not required that the spatial function, e.g., a function from the 3D spatial domain to the real numbers, is a density function, that is, negative values are allowed. The reason for allowing this is that in the course of training, although at one end of the spectrum density functions are sampled corresponding to physical molecules, during training these points are disturbed, so that at the other end of the spectrum the spatial function represented by the points may have devolved to a spatial function rather than a spatial density function. Indeed, the scale indicated in the lower right corner of Figure 3 allows for values up to -1.

In an embodiment, the 0% image is obtained by sampling positions and their features from a standard normal distribution with zero mean.

After generating the initial 0% spatial functions, in the form of a representation of position-value pairs, the denoising network is repeatedly applied. The neural network is trained to modify the positions and/or values of the plurality of points to become closer to the empirical distribution of physical molecules in the training data.

As can be seen in Figure 3, as more iterations are processed, the points start to converge more and more on an actual molecule. Three images of Figure 3a have been produced on a larger scale.

Figure 3b schematically shows an example of an embodiment of an initial noisy spatial density function at 0%

Figure 3c schematically shows an example of an embodiment of intermediate molecular structure at 54%

Figure 3d schematically shows an example of an embodiment of a molecular structure generated before discretization at 100%,

### Training

Figure 2b schematically shows an example of an embodiment of a denoising training system 200.

To train the denoising neural network, molecular structure training data 270 is needed, that is a plurality of molecular structures comprising a plurality of molecular substructure positions. Various public or proprietary datasets may be used. For example, the worldwide protein data bank archive may be used for plurality 270. Furthermore, the QM9 dataset, or GEOM-drug dataset may be used.

The molecular structures in the training set are first converted to spatial density functions. In an embodiment, system 200 comprises a density function conversion unit 280 configured to produce a density function representation 281.

For example, a suitable spatial domain may be chosen, e.g., the unit interval cubed, or the like.

To convert a molecular structure, its molecular substructures, e.g., atoms, first positional information is mapped to the spatial domain, e.g., by applying a scaling factor; the molecular structure is embedded in the spatial domain. Thus obtaining a set of means: µ₁, ... , *µₘ.* A spatial density function is then defined as *f*(*x*) *=* ${\sum }_{i = 1}^{m} N \left(x; {μ}_{i}\right)$*.* Here *N*(*x; µᵢ*) is a distribution with mean *µᵢ*. Typically, a Gaussian with fixed standard deviation, e.g., unit standard deviation is used. Other distributions than Gaussian can be used, e.g., a Gamma distribution. For example, the spatial domain may be a cubed interval of a size proportional to the standard deviation, e.g., using at least 5 times, at least 10 times, etc., the standard deviation, e.g., use 10 if the standard deviation is 1.

The spatial function f is then represented in a data structure, e.g., density function representation 281. For example, a series of values of *f* along a regular grid, e.g., voxels in the spatial domain may be used for representation 281.

Preferably however, a *f* is regarded as a non-normalized probability distribution and sampled according to its distribution. Various approaches exist, e.g., rejection sampling, or mixture sampling, e.g., Gaussian mixture sampling. This produces a list of points *xᵢ*, with preference given to regions with higher density. This is advantageous since regions with higher density are exactly the regions where molecular substructures are located and thus where it is desired to have the most information. For each point the density is computed producing a list of pairs (*xᵢ, f*(*xᵢ*))*.*

Finally, representation 281 is processed by diffusor 290. Diffusor repeatedly applies a disturbance to the elements of representation 281, producing a list of increasingly disturbed spatial function representations 291-293. The number of produced disturbed spatial functions may be equal to the number of training steps performed for a given training element. To disturb a spatial function represented by a regular grid, e.g., a voxel based approach, it is sufficient to disturb the density value, e.g., by adding a value sampled from a Gaussian distribution. To disturb a spatial function represented by a sequence of values both position and density value may be disturbed, e.g., by adding a value sampled from a Gaussian to the density, and adding a value sampled from a Gaussian or Gaussian process to the position.

Once the disturbed spatial function representations are obtained, they are used to train the denoising neural network. The network is trained as a generative model or conditional generative model, learning to predict the original spatial function representation 281 given progressively noisier inputs. A loss function, such as mean squared error (MSE) or a variation of Kullback-Leibler divergence, may be used to quantify the difference between the predicted and ground truth representations. During training, a sequence of corrupted spatial function representations is provided to the network, which learns to iteratively refine them by minimizing the loss. Gradient-based optimization, typically using Adam or another variant of stochastic gradient descent (SGD), is employed to update the model parameters. The network may be trained over multiple epochs with batch normalization, dropout, or other regularization techniques to prevent overfitting. Once training is complete, the model can be used to denoise new spatial function representations by reversing the applied disturbances and reconstructing a molecular structure.

Through the use of spatial functions, e.g., excitation fields, new graphs can be generated that have an arbitrary number of nodes-the number of nodes need not be fixed before generation, though it could be controlled through conditioning data. It is important to note that this generative approach differs from graph neural networks, even graph neural networks configured to work on continuous graph representations, which primarily focus on solving traditional graph-related inference tasks using this generalized graph concept. There are two distinct ways to approach the generative process. The first approach is through field diffusion as shown above, while the second one uses inverse heat dissipation, as further described herein. Note that these two different approaches are used for generating fields, while the graph conversion that we will finally introduce is agnostic to the model used for field generation.

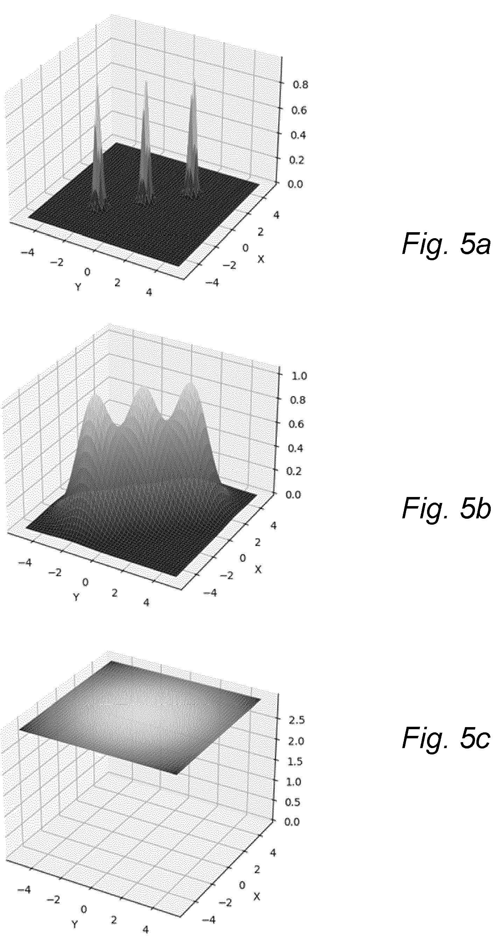

Instead of using a stochastic equation to progressively disperse the spatial density function over time, it is also possible to apply a heat dissipation process to the spatial density function for perturbation. Figure 5a schematically shows an example of an embodiment of a continuous graph representing a molecular structure. A molecule with three atoms has been transformed into a density function, in this case with a two dimensional spatial domain. The graph of Figure 5a can be sampled to obtain a data structure representing it. Figures 5b and 5c schematically show an example of an embodiment of a disturbed continuous graph. The density function of Figure 5a has been disturbed according to a heat dispersion process. In Figure 5b, an intermediate stage of the disturbance process is shown. In Figure 5c, the density function has been fully flattened. Each of the disturbed density functions can be sampled to obtain training data.

It is noted that both the embedding of the original molecular structure in the training data, and the disturbing process can be performed in many ways, e.g., under the control of a random number generator. For example, different positions may be chosen to represent the same molecular structure. For example, different samples may be drawn from a Gaussian distribution to disperse a spatial function in different ways.. In an embodiment, the embedding and/or dispersing is repeated multiple times to enlarge the training set.

### Continuous Graph Neural Networks Computations

Once a discretized molecular structure has been determined, e.g., first molecular structure 241 or second molecular structure 251, various properties can be computed for it. Such computing could be done using a graph neural network.

A Graph Neural Network (GNN) can compute molecular properties by treating the molecular structure as a graph, where atoms are nodes and bonds are edges. The GNN iteratively updates node (atom) and edge (bond) representations through message passing, where each node aggregates information from its neighbors using learnable transformation functions. This process captures local and global structural patterns, enabling the model to encode chemical interactions and spatial dependencies. After multiple message-passing iterations, a graph-level representation is obtained via pooling or readout functions, which can be used for property prediction tasks such as energy levels, reactivity, or solubility.

It would be desirable to apply graph neural network directly on the undiscretized molecular structure. Through the discretizing soft information is lost which may be useful during further computations.

Examples of properties that can be computed using either a discrete or continuous graph neural network computation include: solubility, toxicity, binding affinity, e.g., drug-target interactions-all of these are important drug development, electronic properties, thermodynamic stability, amongst others.

In an embodiment, connectivity between the identified molecular substructure positions is inferred by applying a computational bond determination method to construct a spatial density function indicating a likelihood of a connection existing between two points. For example, for a given spatial domain *D*, the function may be a function from ${ρ}_{e} : D \times D \to {ℝ}^{\geq 0}$. The function gives a likelihood of the presence of a bond between the two positions. In an embodiment, the function *ρₑ*(*x, y*) is constructed as *ρₑ*(*x, y*) *= c*(*x, y*)*ρ*(*x*)*ρ*(*x*)*,* wherein *ρ* is the generated spatial density function. Next one or more noisy feature fields are generated, each noisy feature field being a function from a spatial domain to real numbers. The noisy feature fields could be generated before the denoising and updated during the denoising. The noisy feature fields could instead be generated after the denoising. In both cased the one or more feature fields are iteratively updated in a process after the denoising. The process comprising integrating over the spatial density function indicating the likelihood of connection existing, the one or more noisy feature fields, and a trained function. The function *ρₑ* may instead by approximated by a function that exponentially decays as a function of the distances between the two input points.

We define operations on these fields and prove their convergence. We now demonstrate how our approach can be applied to tasks commonly tackled by graph neural networks (GNNs). These tasks include graph-level predictions, such as estimating the energy of a molecule, as well as node-level tasks, for example classifying individual nodes. Embodiments are capable of handling these tasks effectively.

Instead of using the process on molecular structures produced through denoising, it can also be applied by converting a discrete graph into a continuous representation.

Assuming we have a field feature at layer k, denoted as **h**^{(*k*)}(**r**), we propose the following operation to calculate the field feature at the next layer: ${h}^{\left(k+1\right)} \left({r}_{i}\right) = W \int {h}^{\left(k\right)} \left({r}_{j}\right) {ρ}_{e} \left({r}_{i}, {r}_{j}\right) {dr}_{j} ,$

Here, **W** represents a linear pointwise transformation. We can understand this convolutional update as a two-step process. First, we perform a convolutional step where we aggregate neighboring spatial information through the integral operation, and then we pointwise transform features using the linear transformation **W.** This can be thought of as an operation using which each point **r***ᵢ* aggregates the features from all other spatial domains, and the contribution is weighed by the density of that point in space having an edge with the point rᵢ. Note that for simplicity, we have omitted the nonlinearity applied after the linear transformation. In 4 we prove that this converges to regular message passing in the limit of

### Continuous graph predictions

Suppose we have performed *K* layers of continuous graph convolutions, and we now want to obtain the feature value associated with each node. For example, this may be done for tasks like node classification or obtaining each node's feature representation. We propose the following method:
The final node representation for node *i* after *K* layers, denoted as ${h}_{i}^{\left(K\right)}$, can be obtained from the feature field **h**^{(*K*)}(**r**) using the following integral: ${h}_{i}^{\left(K\right)} = \int {h}^{\left(K\right)} \left(r\right) {ρ}_{{n}_{i}} \left(r\right) dr$

Here, *ρ_{nᵢ}*(**r**) corresponds to the density distribution for the *i*-th node. If we view the total node density function *ρₙ* as a mixture model, this operation can be interpreted as the expected value of the feature field for the *i*-th component of the mixture. With these node-level vectors, we can proceed with predictions as one typically would in a (GNN).

On the other hand, if we wish to predict a graph-level feature H (equivalent to global pooling in GNNs), we have two options. We can either sum-pool the feature vectors obtained using the previous equations. However, since the model is a mixture model, a linear property holds: $H = {\sum }_{i} {h}_{i}^{\left(K\right)} = {\sum }_{i} \int {h}^{\left(K\right)} \left(r\right) {ρ}_{{n}_{i}} \left(r\right) dr = \int {h}^{\left(K\right)} \left(r\right) {\sum }_{i} {ρ}_{{n}_{i}} \left(r\right) dr = \int {h}^{\left(K\right)} \left(r\right) {ρ}_{n} \left(r\right) dr ,$

In other words, we can view the graph-level feature as the expected value of the feature field.

The inventors realized that computing on continuous representations of graph has utility independent of how the continuous representation is obtained. The following clauses represent useful embodiments.

Clause 1. A computer-implemented method (600) for performing computations on molecular structures, a molecular structure comprising multiple molecular substructures,
the method comprising:
- obtaining a first spatial density function and a second spatial density function representing a molecular structure, wherein the first spatial density function is from a spatial domain to non-negative numbers indicating a likelihood a molecular substructure is present, and the second spatial density function is from the spatial domain squared to non-negative numbers indicating a likelihood a connection, e.g., a bond, exists between two points in the spatial domain,
- generating one or more noisy feature fields, each noisy feature field being a function from a spatial domain to real numbers;
- iteratively updating the one or more feature fields by integrating over the spatial density function indicating the likelihood of connection existing, the one or more noisy feature fields, and a trained function.

Clause 2. The method according to Clause 1, wherein
- the first and/or second spatial density function is represented by sampling at a regular grid, or
- the first and/or second spatial function is represented as a series of positions of a plurality of points, and/or pairs of points and values of the spatial function.

It is noted that any one of the clauses 1 and 2 may be combined with features and embodiments as described herein, e.g., in the description and/or claims.

In our method, feature fields are treated as continuous objects. However, representing and performing operations on these continuous objects within a neural network setting is not simple. Moreover, executing operations like integral transforms, as proposed in the previous subsection, is non-trivial. One approach is to discretize the grid and work with discrete representations of these objects, but this comes at the cost of losing some of the advantageous properties of continuous representations.

Fortunately, we've observed that for translation-invariant convolutions, continuous convolutions effectively become a standard convolution operations. This enables us to perform these operations in the Fourier domain, which offers several advantages. Firstly, these operations are resolution-independent. In other words, if we learn transformations, as we do in methods such as Fourier Neural Operators (FNO), the learned kernels in the Fourier domain remain invariant to the initial discretization of our representations. This strikes a balance between continuous and discretized approaches, allowing us to use the benefits of both approaches. It is possible to train a model that is invariant to discretization.

For translation-invariant kernels, we show that these operations can be efficiently implemented in the Fourier domain, enabling resolution-independent learning. Our method allows for bidirectional conversion between discrete graphs and continuous representations, facilitating both node-level and graph-level predictions.

### Convergence to Message Passing

To verify whether the proposed graph neural network convolution operation converges to the standard message passing equation when the edge functions correspond to those of a regular graph, we can examine the expression step by step: $\begin{matrix}{h}^{\left(k+1\right)} \left({r}_{i}\right) = W \int {h}^{\left(k\right)} \left({r}_{j}\right) {ρ}_{e} \left({r}_{i}, {r}_{j}\right) d {r}_{j} \\ = W \int {\sum }_{i ′ = 1}^{N} {\sum }_{j ′ = 1}^{N} δ \left({r}_{i}-{r}_{i ′}\right) δ \left({r}_{j}-{r}_{j ′}\right) {h}^{\left(k\right)} \left({r}_{j}\right) {e}_{i ′ j ′} d {r}_{j} \\ = W {\sum }_{i ′ = 1}^{N} {\sum }_{j ′ = 1}^{N} δ \left({r}_{i}-{r}_{i ′}\right) {h}^{\left(k\right)} \left({r}_{j ′}\right) {e}_{i ′ j ′} \\ = {\sum }_{i ′ = 1}^{N} δ \left({r}_{i}-{r}_{i ′}\right) \left({\sum }_{j ′ = 1}^{N} W {h}^{\left(k\right)} \left({r}_{j ′}\right) {e}_{i ′ j ′}\right)\end{matrix}$

To understand this expression, note that the first sum over *i'* ensures that we consider node features at all valid locations. The term within the brackets represents the typical aggregation step of the transformed features of neighboring nodes. Therefore, this is equivalent to the graph convolutions observed in regular graphs.

### Decomposed Convolutions

After decomposing the edge function, the convolution operation becomes: $\begin{matrix}{h}^{\left(k+1\right)} \left({r}_{i}\right) = W \int {h}^{\left(k\right)} \left({r}_{j}\right) {ρ}_{e} \left({r}_{i}, {r}_{j}\right) d {r}_{j} \\ = W \int {h}^{\left(k\right)} \left({r}_{j}\right) {ρ}_{n} \left({r}_{i}\right) {ρ}_{n} \left({r}_{j}\right) f \left({r}_{i}, {r}_{j}\right) d {r}_{j} \\ = W \int {h}^{\left(k\right)} \left({r}_{j}\right) {ρ}_{n} \left({r}_{i}\right) f \left({r}_{i}, {r}_{j}\right) {ρ}_{n} \left({r}_{j}\right) d {r}_{j}\end{matrix}$

If we first perform the integral transformation and then apply the pointwise transformation (along with the nonlinearity which is omitted here), the integral from the previous equation would have the form: ${h}^{\left(k+1\right)} \left({r}_{i}\right) = \int {h}^{\left(k\right)} \left({r}_{j}\right) {ρ}_{n} \left({r}_{i}\right) f \left({r}_{i}, {r}_{j}\right) {ρ}_{n} \left({r}_{j}\right) d {r}_{j} = \int {h}^{\left(k\right)} \left({r}_{j}\right) K \left({r}_{i}, {r}_{j}\right) d {r}_{j}$

In this order, we can recognize that this is equivalent to an integral transform with the kernel *K*(**r***ᵢ,* **r***ⱼ*) = *ρₙ*(r*ᵢ*)*f*(**r***ᵢ,***r***ⱼ*)*ρₙ*(**r***ⱼ*)*.*

### Translation-invariant Convolutions

We can make further assumptions on the function *f*(**r***ᵢ*, **r***ⱼ*). Specifically, one may assume that it can be written in a translation-invariant form $f \left({r}_{i}, {r}_{j}\right) = f \left(0,{r}_{i}-{r}_{j}\right) ≡ f \left({r}_{i}-{r}_{j}\right) .$

For example, the physically inspired function, would naturally fall into this category. With this assumption, the integral becomes: $\begin{matrix}{h}^{\left(k+1\right)} \left({r}_{i}\right) = \int {h}^{\left(k\right)} \left({r}_{j}\right) {ρ}_{n} \left({r}_{i}\right) f \left({r}_{i}-{r}_{j}\right) {ρ}_{n} \left({r}_{j}\right) d {r}_{j} \\ = {ρ}_{n} \left({r}_{i}\right) \int {h}^{\left(k\right)} \left({r}_{j}\right) f \left({r}_{i}-{r}_{j}\right) {ρ}_{n} \left({r}_{j}\right) d {r}_{j} \\ = {ρ}_{n} \left({r}_{i}\right) \int {u}^{\left(k\right)} \left({r}_{j}\right) f \left({r}_{i}-{r}_{j}\right) d {r}_{j} ,\end{matrix}$ with **u**^{(*k*)}(**r***ⱼ*) ≡ **h**^{(*k*)}(**r***ⱼ*)*ρₙ*(**r***ⱼ*)*.* We can recognize that this is a convolution between functions **u** and *f*. The latter makes the approach more practical, since it allows computation using the Fourier transform, e.g., FFT, which is faster.

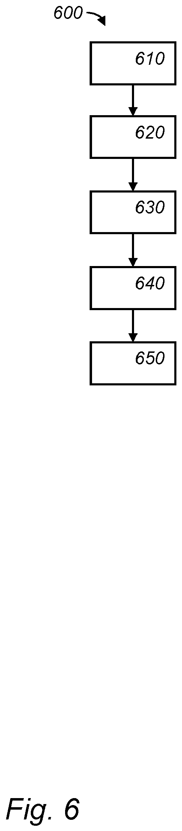

Figure 6 schematically shows an example of an embodiment of a method (600) for generating molecular structures. Method 600 may be computer-implemented and comprises
- generating (610) an initial noisy spatial function and representing the spatial function in a data structure;
- progressively (620) denoising the spatial function by iteratively applying a trained neural network, wherein the neural network is trained to transform noisy density functions into structured spatial density functions based on training data comprising molecular structures represented as spatial density functions,
- computing (630) an integral of the spatial density function obtained from the denoising and determining an integer value based on the result;
- fitting (640) a sum of a specified number of predefined density distributions, wherein the specified number corresponds to the determined integer value;
- identifying (650) centers of the fitted density distributions as positions of the molecular substructures of the molecular structure.

For example, the data structure representing the spatial function, parameters, e.g., weights, representing the neural network, and identified centers of the fitted density distributions may be stored in an electronic storage, e.g., a memory, a hard drive, etc. For example, applying a neural network to the spatial function, during generation or training, and/or adjusting the stored parameters to train the network may be done using an electronic computing device, e.g., a computer.

The neural networks, either during training and/or during applying may have multiple layers, which may include, e.g., convolutional layers and the like. For example, the neural network may have at least 2, 5, 10, 15, 20 or 40 hidden layers, or more, etc. The number of neurons in the neural network may, e.g., be at least 10, 100, 1000, 10000, 100000, 1000000, or more, etc.

Many different ways of executing the method are possible, as will be apparent to a person skilled in the art. For example, the steps can be performed in the shown order, but the order of the steps can be varied, or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing one or more computers, e.g., a processor system, to perform an embodiment of method 600. The software may include only those steps taken by a particular sub-entity of the system. The software and/or other data according to an embodiment may be stored in a non-transitory storage medium, such as a hard disk, a floppy disk, a memory, an optical disc, read-only memory, random access memory, CD-ROMs, magnetic tape, optical data storage devices, etc. Transitory signals and carrier waves are excluded from non-transitory media.

The software may be sent as a transitory signal along a wire or wirelessly, e.g., sent as a transitory signal over a data network, e.g., the Internet. For example, signals and/or carrier waves may serve as a transitory medium for carrying information. For example, a modulated electromagnetic wave may carry a signal bearing the software and/or other data according to an embodiment.

The software may be made available for download and/or for remote usage on a server. Embodiments of the method may be executed using a bitstream arranged to configure programmable logic, e.g., a field-programmable gate array (FPGA), to perform an embodiment of the method.

It will be appreciated that the presently disclosed subject matter also extends to computer programs, particularly computer programs on or in a carrier, adapted for putting the presently disclosed subject matter into practice. The program may be in the form of source code, object code, code intermediate between source and object code, such as partially compiled code, or in any other form suitable for use in the implementation of an embodiment of the method. An embodiment relating to a computer program product comprises computer-executable instructions corresponding to each of the processing steps of at least one of the methods set forth. These instructions may be subdivided into subroutines and/or be stored in one or more files that may be linked statically or dynamically. Another embodiment relating to a computer program product comprises computer-executable instructions corresponding to each of the devices, units, and/or parts of at least one of the systems and/or products set forth.

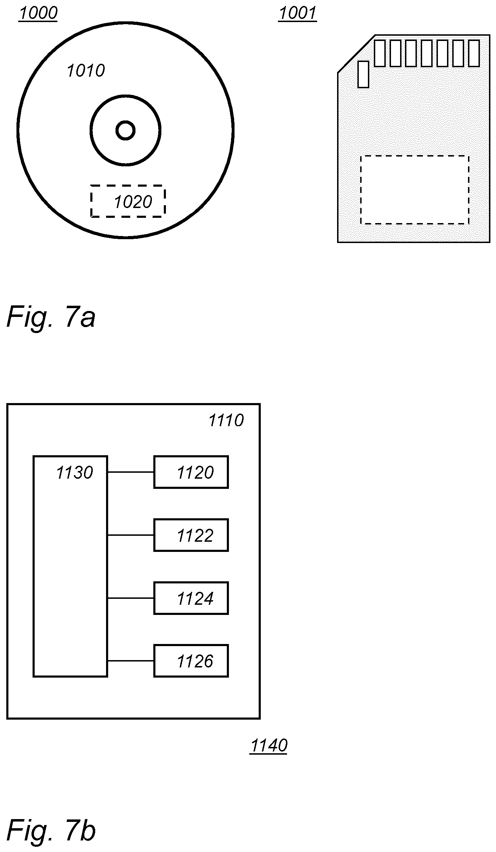

Figure 7a shows a computer-readable medium 1000 having a writable part 1010, and a computer-readable medium 1001 also having a writable part. Computer-readable medium 1000 is shown in the form of an optically readable medium. Computer-readable medium 1001 is shown in the form of an electronic memory, in this case a memory card. Computer-readable mediums 1000 and 1001 may store data 1020 wherein the data may indicate instructions which, when executed by a processor system, cause a processor system to perform an embodiment of a method for generating molecular structures and/or a method for training a neural network to transform noisy spatial functions into structured spatial density functions. The computer program 1020 may be embodied on the computer-readable medium 1000 as physical marks or by magnetization of the computer-readable medium 1000. However, any other suitable embodiment is conceivable as well. Furthermore, it will be appreciated that, although the computer-readable medium 1000 is shown here as an optical disc, the computer-readable medium 1000 may be any suitable computer-readable medium, such as a hard disk, solid-state memory, flash memory, etc., and may be non-recordable or recordable. The computer program 1020 comprises instructions for causing a processor system to perform an embodiment of said method for generating molecular structures and/or said method for training a neural network to transform noisy spatial functions into structured spatial density functions.

Figure 7b shows a schematic representation of a processor system 1140 according to an embodiment. The processor system comprises one or more integrated circuits 1110. The architecture of the one or more integrated circuits 1110 is schematically shown in Figure 7b. Integrated circuits 1110 comprises a processing unit 1120, e.g., a processor, a CPU, for running computer program components to execute a method according to an embodiment and/or implement its modules or units. Integrated circuits 1110 comprises a memory 1122 for storing programming code, data, etc. Part of memory 1122 may be read-only. Integrated circuits 1110 may comprise a communication element 1126, e.g., an antenna, connectors, or both, and the like. Integrated circuits 1110 may comprise a dedicated integrated circuit 1124 for performing part or all of the processing defined in the method. Processing unit 1120, memory 1122, dedicated IC 1124 and communication element 1126 may be connected to each other via an interconnect 1130, such as a bus. The processor system 1140 may be arranged for contact and/or contactless communication, using an antenna and/or connectors, respectively.

For example, in an embodiment, processor system 1140, e.g., the molecular structure generating system or device, and/or training system or device for training a neural network to transform noisy spatial functions into structured spatial density functions, may comprise a processor circuit and a memory circuit, the processor being arranged to execute software stored in the memory circuit. The memory circuit may be a ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While system 1140 is shown as including one of each described component, the various components may be duplicated in various embodiments. For example, the processing unit 1120 may include multiple microprocessors that are configured to independently execute the methods described herein or are configured to perform elements or subroutines of the methods described herein such that the multiple processors cooperate to achieve the functionality described herein. Further, where the system 1140 is implemented in a cloud computing system, the various hardware components may belong to separate physical systems. For example, the processing unit 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of" when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A computer-implemented method (600) for generating molecular structures, a generated molecular structure comprising a plurality of molecular substructure positions, the method comprising:
- generating (610) an initial noisy spatial density function and representing the spatial function in a data structure;
- progressively (620) denoising the spatial function by iteratively applying a trained neural network, wherein the neural network is trained to transform noisy density functions into structured spatial density functions based on training data comprising molecular structures represented as spatial density functions,
- computing (630) an integral of the spatial density function obtained from the denoising and determining an integer value based on the result;
- fitting (640) a sum of a specified number of predefined density distributions, wherein the specified number corresponds to the determined integer value;
- identifying (650) centers of the fitted density distributions as positions of the molecular substructures of the molecular structure.

2. A method as in Claim 1, wherein
- a noisy spatial density function is configured to represent an arbitrary number of molecular substructures positions, and/or
- the trained neural network is configured to increase and/or decrease an integral of the spatial function in a denoising iteration.

3. A method as in any one of the preceding claims, comprising
- inferring connectivity between identified molecular substructure positions by applying a computational bond determination method to construct a molecular graph.

4. The method according to any one of the preceding claims, further comprising:
- generating one or more noisy feature fields, each feature field being a function from a spatial domain to real numbers or vector of real numbers;
- progressively denoising the one or more noisy feature fields by iteratively applying the trained neural network;
- sampling the one or more denoised feature fields at the identified positions of the molecular substructures to obtain feature values associated with each molecular substructure.

5. The method of Claim 4, wherein the feature values represent one or more of: atom type, charge, electronegativity, partial atomic charge, hybridization state, van der Waals radius, bonding preferences, and polarizability.

6. The method of any one of the preceding claims, wherein the spatial density function is defined over a 2- or 3-dimensional space.

7. The method of Claim 3, comprising applying a graph neural network to the molecular graph to predict molecular properties.

8. The method according to any one of the preceding claims, comprising
- inferring connectivity between the identified molecular substructure positions by applying a computational bond determination method to construct a spatial density function indicating a likelihood of a connection existing between two points,
- generating one or more noisy feature fields, each noisy feature field being a function from a spatial domain to real numbers;
- iteratively updating the one or more feature fields by integrating over the spatial density function indicating the likelihood of connection existing, the one or more noisy feature fields, and a trained function.

9. The method according to any one of the preceding claims, wherein
- the spatial density function is represented by sampling at a regular grid, or
- the spatial function is represented as a series of positions of a plurality of points, and values of the spatial function, and wherein the trained neural network is configured to modify the positions and/or values of the plurality of points.

10. The method of any one of the preceding claims, wherein the generation of the molecular structure is conditioned on one or more conditioning parameters, the method further comprising:
- receiving one or more conditioning parameters specifying desired molecular properties or structural constraints;
- modifying the initial noisy spatial function using the one or more conditioning parameters and/or providing the one or more conditioning parameters to the trained neural network when applied to the spatial function, wherein the neural network is trained to transform noisy spatial functions into structured spatial density functions conditioned by the one or more conditioning parameters.

11. The method of Claim 10, wherein the one or more conditioning parameters comprise experimental data (e.g., comprising full or partial NMR spectra, mass spectra, MS fragmentation), wherein the method is configured to generate a molecular structure corresponding to the experimental data.

12. The method according to any one of the preceding claims, comprising
- generating a plurality of molecular structures according to the method according to any one of the preceding claims,
- applying a further chemical software, configured to receive a molecular structure, to the plurality of molecular structures to test, debug, stress-test, or benchmark the further chemical software

13. The method according to any one of the preceding claims, comprising applying a retrosynthesis tool to the molecular structure, obtaining a synthetic route, and synthesizing a molecule according to the molecular structure.

14. A computer-implemented method for training a neural network to transform noisy spatial functions into structured spatial density functions, the method comprising
- obtaining a plurality of molecular structures comprising a plurality of molecular substructure positions, the molecular structures corresponding to structures of physical molecules,
- representing the molecular structures as spatial density functions, by adding a number of predefined density distributions fitted to the plurality of molecular substructure positions, wherein the number corresponds to the number of molecular substructures in the plurality of molecular substructures, and wherein means of the fitted density distributions correspond to positions of the molecular substructures,
- generating diffusion training data by progressively applying a noise perturbation process to the spatial density function, wherein the noise perturbation process iteratively transforms the spatial density function into a noisy spatial function;
- training the neural network using the diffusion training data, wherein the neural network is trained to reconstruct the original spatial density function by minimizing a loss function that penalizes deviations between the denoised spatial function and the original structured spatial density function.

15. The method of Claim 14, wherein
- the noise perturbation process comprises applying a diffusion process to the spatial density function, wherein the diffusion process is governed by a stochastic equation that progressively disperses the spatial density function over time, and/or
- the noise perturbation process comprises applying a heat dissipation process to the spatial density function.

16. One or more non-transitory computer-readable media and/or one or more transitory computer-readable media storing computer-executable instructions that, when executed by a computing system, cause the computing system to perform the method according to any one of the preceding claims.

17. A system comprising: one or more processors; and one or more storage devices storing instructions that, when executed by the one or more processors, cause the one or more processors to perform operations according to the method according to any one of the preceding claims.
